# EUROPÄISCHE PATENTSCHRIFT

(11) **EP 0 266 563 B1**
(45) Veröffentlichungstag und Bekanntmachung des Hinweises auf die Patenterteilung: **29.01.1992**
(21) Anmeldenummer: 87114443.2
(22) Anmeldetag: 03.10.1987
(51) Int. Cl.: A61K 31/40, A61K 31/415, A61K 31/42, A61K 31/44, A61K 31/445, A61K 31/495, A61K 31/535

(54) **Verwendung von 1-Benzyl-amino-alkyl-pyrrolidinonen als Antidepressiva**
Use of 1-benzyl-amino-alkyl-pyrrolidinones as antidepressants
Utilisation de 1-benzyl-amino-alkyl-pyrrolidinones comme antidépresseurs

(30) Priorität: 08.10.1986 DE 3634220
(43) Veröffentlichungstag der Anmeldung: 11.05.1988
(73) Patentinhaber: BOEHRINGER INGELHEIM KG, 55216 Ingelheim (DE); BOEHRINGER INGELHEIM INTERNATIONAL GmbH, 55218 Ingelheim am Rhein (DE)
(72) Erfinder: Lehr, Erich, Dr., D-6531 Waldalgesheim (DE); Bechtel, Wolf-Dietrich, Dr., D-6531 Appenheim (DE); Böke-Kuhn, Karin, Dr., D-6535 Gau-Algesheim (DE); Schneider, Claus, Dr., D-6507 Ingelheim am Rhein (DE); Walther, Gerhard, Dr., D-6530 Bingen am Rhein (DE); Weber, Karl-Heinz, Dr., D-6535 Gau-Algesheim (DE)

(56) Entgegenhaltungen:
- EP-A- 0 136 658
- J. INT. MED. RES., Band 7, Nr. 4,1979, Seiten 277-284, Cambridge medical Publications Limited; J. KABES et al.: "Biological correlates of piracetam clinical effects in psychotic patients" *seite 279, rechte spalte, Zeile 1- seite 283, linke spalte, Zeile 26
- MUNCH. MED. WSCHR., Band 118, nr. 29/30, 1976, Seiten 957-958; F. ECKMANN: "Klinische Untersuchungen mit Piracetam" * Insgesamt *
- FARMACOL. TOKSICOL, Band 48, Nr. 2, 1985, Seiten 32-25; N.N. KARKISHCHENKO et al.: "Comparative study of some parameters of antidepressant activity of potassium orotate and piracetam"
- PSYCHOPHARMACOLOGY, Band 97, Nr. 2, 1989, Seiten 145-146, Springer-Verlag; F. LEHR: "Distress call reactivation in isolated chicks: a behavioral indicator with high selectivity for antidepressants"

## Beschreibung

Die Erfindung betrifft die Verwendung von 1-Benzyl-aminoalkyl-pyrrolidinonen der allgemeinen Formel 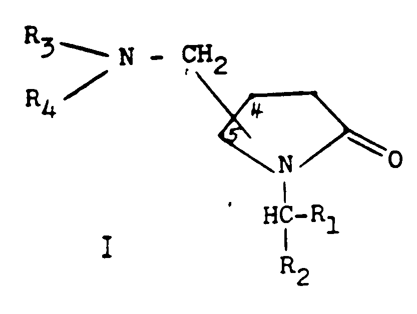
worin
- R₁: Wasserstoff oder einen Alkylrest,
- R₂: einen Phenylrest, der ein- oder zweifach durch Alkoxy, Fluor, Chlor, Brom, Trifluormethyl, Alkyl, Hydroxy oder Nitro substituiert sein kann oder einen Pyridylrest,
- R₃: Wasserstoff oder einen Alkylrest und
- R₄: Wasserstoff oder einen Alkylrest bedeuten können oder beide Reste R₃ und R₄ zusammen mit dem Stickstoffatom einen gesättigten 5- oder 6-Ring, der als weiteres Heteroatom ein O- oder N-Atom enthalten kann und gegebenenfalls durch Methyl substituiert sein kann oder einen Imidazolring bilden und wobei die Aminoalkylgruppe in 4- oder 5-Stellung steht und deren pharmakologisch verträgliche Säureadditonssalze,
als Antidepressiva.

In der allgemeinen Formel I steht die Bezeichnung "Alkyl" für eine geradkettige oder verzweigte Alkylgruppe mit 1 -4 Kohlenstoffatomen, wie z.B. Methyl, Ethyl, Propyl, Isopropyl, n-Butyl oder tert.-Butyl und die Bezeichnung "Alkoxy" für einen Rest mit 1 - 2 Kohlenstoffatomen; der als Bedeutung für R₂ genannte Pyridylring kann in 2-, 3-oder 4-Stellung mit der Methylenbrücke verknüpft sein. Bevorzugte Alkylgruppen sind Methyl und Ethyl.

Verbindungen der allgemeinen Formel I und Verfahren zu ihrer Herstellung sind aus der europäischen Patentanmeldung 136 658 bekannt, wobei die Wirksamkeit der Verbindungen bei Zustandsformen eingeschränkter cerebraler Leistungsfähigkeit offenbart wird.

Überraschenderweise hat sich gezeigt, daß die Verbindungen der allgemeinen Formel I gut wirksame Antidepressiva eines neuen Strukturtyps darstellen.

Bevorzugt sind Verbindungen der allgemeinen Formel I, bei denen R₁ Wasserstoff, R₂ einen gegebenenfalls durch Fluor, Chlor, Methyl oder Methoxy in o- oder p-Stellung substituierten Phenylrest und R₃ und R₄ Wasserstoff, Methyl oder Ethyl oder R₃ und R₄ zusammen Morpholin-, N-Methylpiperazin oder Pyrrolidin bedeuten.

Geeignete Verbindungen sind weiterhin:
1-(3,4-Dimethoxybenzyl)-4-aminomethyl-pyrrolidin-2-on
1-(4-Methylbenzyl)-4-aminomethyl-pyrrolidin-2-on
1-(3-Trifluormethylbenzyl)-4-aminomethyl-pyrrolidin-2-on
1-(α-Methylbenzyl)-4-aminomethyl-pyrrolidin-2-on
1-Benzyl-4-piperidinomethyl-pyrrolidin-2-on
1-Benzyl-4-(N-methylpiperazinylmethyl)-pyrrolidin-2-on
1-Benzyl-4-(imidazol-1-yl-methyl)-pyrrolidin-2-on
1-Benzyl-4-methylaminomethyl-pyrrolidin-2-on
1-(p-Fluorbenzyl)-4-dimethylaminomethyl-pyrrolidin-2-on
1-(4-Nitrobenzyl)-4-aminomethyl-pyrrolidin-2-on
1-(4-Hydroxybenzyl)-4-aminomethyl-pyrrolidin-2-on
1-(o-Chlorbenzyl)-4-aminomethyl-pyrrolidin-2-on
1-(o-Chlorbenzyl)-4-diethylaminomethyl-pyrrolidin-2-on
1-Benzyl-4-isopropylaminomethyl-pyrrolidin-2-on
1-(p-Methylbenzyl)-4-diethylaminomethyl-pyrrolidin-2-on
1-Benzyl-5-dimethylaminomethyl-pyrrolidin-2-on
1-Benzyl-5-morpholinomethyl-pyrrolidin-2-on
1-Benzyl-5-(4-methylpiperazino)-methyl-pyrrolidin-2-on
1-(4-Methylbenzyl)-5-dimethylaminomethyl-pyrrolidin-2-on
1-(4-Methylbenzyl)-5-diethylaminomethyl-pyrrolidin-2-on
1-(p-Chlorbenzyl)-5-diethylaminomethyl-pyrrolidin-2-on
1-(3,4-Dichlorbenzyl)-5-dimethylaminomethyl-pyrrolidin-2-on
1-(3,4-Dichlorbenzyl)-5-diethylaminomethyl-pyrrolidin-2-on
1-(p-Methoxybenzyl-5-dimethylaminomethyl-pyrrolidin-2-on
1-(p-Methoxybenzyl)-5-diethylaminomethyl-pyrrolidin-2-on
1-Benzyl-5-aminomethyl-pyrrolidin-2-on

Bevorzugte Verbindungen sind:
1-(4-Methoxybenzyl)-4-aminomethyl-pyrrolidin-2-on
1-Benzyl-4-N,N-diethylaminomethyl-pyrrolidin-2-on
1-(4-Fluorbenzyl)-4-aminomethyl-pyrrolidin-2-on
1-(4-Chlorbenzyl)-4-aminomethyl-pyrrolidin-2-on
1-(4-Pyridylmethyl)-4-aminomethyl-pyrrolidin-2-on
1-(4-Fluorbenzyl)-4-(morpholinomethyl)-pyrrolidin-2-on
1-Benzyl-4-(N-methylpiperazinylmethyl)-pyrrolidin-2-on
1-Benzyl-4-methylaminomethyl-pyrrolidin-2- on.

Ganz besonders bevorzugt ist
1-Benzyl-4-aminomethyl-pyrrolidin-2-on
1-Benzyl-5-diethylaminomethyl-pyrrolidin-2-on
1-Benzyl-5-pyrrolidinomethyl-pyrrolidin-2-on
1-(p-Chlorbenzyl)-5-dimethylaminomethyl-pyrrolidin-2-on

Ein empfindlicher Test für den praeklinischen Wirkungsnachweis antidepressiver Eigenschaften ist der Küken-Ruftest. Dabei gilt die im Testverlauf abnehmende Rufhäufigkeit isolierter Eintagsküken als tierexperimentelles Verhaltensmodell zu Resignationserscheinungen bei der Depression. Das Verfahren konnte mit dem Test zahlreicher neurotrop wirksamer Substanzen validiert werden; es zeichnet sich durch gut reproduzierbare Selektivität für bereits klinisch bewährte Antidepressiva aus, welche dosisabhängig die abgesunkene Rufrate zu reaktivieren vermögen (Distress call activation in isolated chicks; A new behavioural model for antidepressants, E. Lehr, Psychopharmacol. 89, 21 (1986); Aktivierung des Kontaktrufens als tierexperimentelles Verhaltensmodell zur Depressionsforschung, E. Lehr, Fortschr. Neurol. Psychiat. 54, 26 (1986).

In Tabelle I sind die pharmakologischen Daten für 1-Benzyl-4-aminomethyl-pyrrolidin-2-on (Fumarat) [Verbindung A] aufgeführt. Im Vergleich dazu sind die entsprechenden Werte von 1-Acetamido-2-pyrrolidinon [Verbindung B], einem strukturell ähnlich gebauten Nootropicum, aufgeführt.

| Tab. I | Verb. A | Verb. B |
|---|---|---|
| Küken-Ruftest ED₁₅₀, mg/kg i.p. | 40 | >> 160 |
| Tetrabenazin-Antagonismus (Ptosis-Aufhebung, Maus) | 110 | >> 640 |
| LD₅₀, Maus mg/kg oral | >> 2000 | |
| Einfluß auf die cholinerge Funktion | Förderung | keine |
| Rezeptor-Bindungen (adrenerg, serotonerg) | keine | keine |
| IC₅₀ [10⁻⁹ Mol/ltr.] | > 10 000 | |

In der Tabelle II sind die pharmakologischen Daten des Kükenruftests einiger Verbindungen der allgemeinen Formel I wiedergegeben.

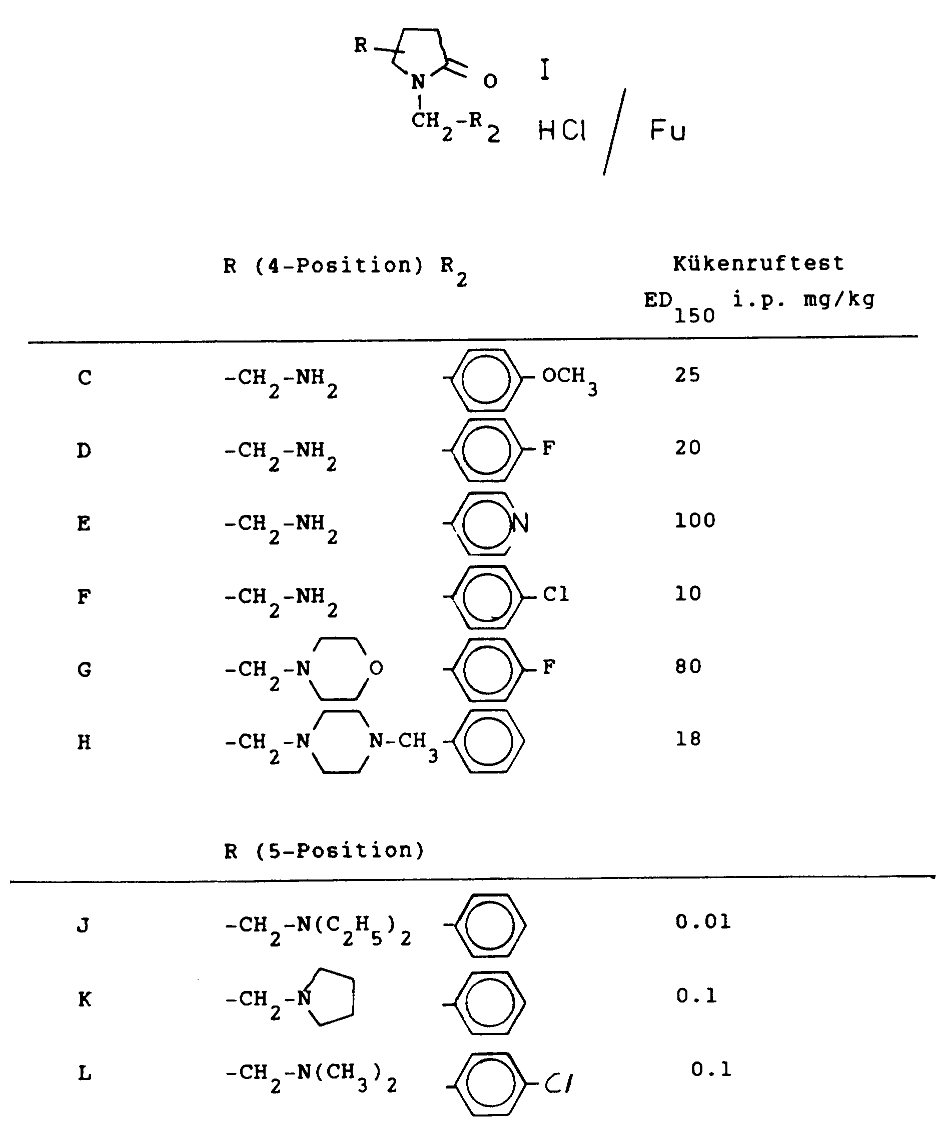

Aufgrund der wiedergegebenen Daten lassen sich eindeutig präklinisch antidepressive Wirkeigenschaften nachweisen, ohne daß die erfindungsgemäßen Verbindungen die typischen Nebenwirkungen herkömmlicher Antidepressiva, wie z.B. Sedation, aufweisen. Das Fehlen von Rezeptor-Bindungseigenschaften und/bzw. Wiederaufnahme-Hemmung biogener Amine deutet auf einen völlig neuartigen Wirkungsmechanismus für antidepressiv wirkende Verbindungen hin. Die cholinomimetische Eigenschaft der Verbindungen der allgemeinen Formel I schließt jene Kardiotioxizität aus, die bei herkömmlichen Antidepassiva durch deren anticholinerge Nebenwirkung verursacht wird und zu den stärksten unerwünschten Nachteilen derartiger Medikamente zählt. Aufgrund der cholinergen Eigenschaft der Verbindungen der allgemeinen Formel I ist der Einsatz der Verbindungen bei Depressionen auch bei geriatrischen Patienten ermöglicht, bei deren cholinerger Mangelfunktion herkömmliche Antidepressiva infolge ihrer anticholinergen Nebenwirkung kontraindiziert sind.

Verfahren zur Herstellung von Verbindungen der allgemeinen Formel I sowie ihrer pharmakologisch verträglichen Säuredadditonssalze sind in der europäischen Patentanmeldung 136 658 beschrieben, auf die hiermit inhaltlich Bezug genommen wird.

Die Verbindungen der allgemeinen Formel I können allein oder in Kombination mit anderen erfindungsgemäßen Wirkstoffen, gegebenenfalls auch in Kombination mit weiteren pharmakologisch aktiven Wirkstoffen zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk und/oder Mitteln zur Erzielung des Depoteffektes, wie Carboxypolymethyylen, Carboxymethylcellulose, Celluloseacetatphthalat, oder Polyvinylacetat erhalten werden. Die Tabletten können auch aus mehreren Schichten bestehen.
Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Äthylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen oder Äthylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Die Dosierung einer therapeutisch wirksamen Menge liegt im allgemeinen zwischen 1 und 150 mg, bevorzugt zwischen 50 und 100 mg je Einzeldosis.

Die nachfolgenden Beispiele illustrieren die vorliegende Erfindung, ohne sie jedoch in ihrem Umfang zu beschränken:

### Beispiel 1

### 1-Benzyl-4-aminomethyl-pyrrolidin-2-on

54 g (0,16 Mol) 4-Phthalimidomethyl-1-benzylpyrrolidin-2-on werden in 1,3 l Äthylalkohol nach Zusatz von 32 g Hydrazinhydrat 4 Stunden bei Raumtemperatur gerührt. Der Niederschlag (Phthalsäurehydrazid) wird abgesaugt und das Filtrat eingedampft. Man setzt 500 ml Methylenchlorid zu dem Rückstand und schüttelt dreimal mit 100 ml Wasser aus. Die organische Phase wird getrocknet und eingedampft. Den verbleibenden Rückstand löst man in 500 ml Methanol und fügt bei Siedehitze unter Rühren 20 g (0,17 Mol) feste Fumarsäure portionsweise hinzu. Beim Abkühlen scheiden sich farblose Kristalle ab, die man absaugt und mit Methanol und Äther nachwäscht.
Ausbeute: 20 - 25 g (48 - 60 % d.Th.)
vom Fp. 209 - 211°C.
Die Verbindung enthält 1/2 Mol Fumarsäure.

Das Ausgangsmaterial wird wie folgt erhalten:
a) 94 g (0,46 Mol) 1-Benzyl-4-hydroxymethyl-pyrrolidin-2-on werden wird 700 ml Methylenchlorid und 40 ml (0,54 Mol) Thionylchlorid 25 Stunden unter Rückflußkochen gerührt und das Reaktionsgemisch anschließend unter Kühlung mit verdünntem Ammoniak neutralisiert. Nach Abtrennen, Trocknen und Eindampfen hinterbleiben 85 - 90 g eines dunkeln Öls, welches direkt zur weiteren Umsetzung verwendet wird.
b) 43,5 g (0,195 Mol) rohes 1-Benzyl-4-chlormethylpyrrolidin-2-on, 36 g (0,195 Mol) Phthalimidkalium und 700 ml Dimethylformamid werden 2 Stunden unter Rückfluß gekocht. Das Reaktionsgemisch wird anschließend im Vakuum eingedampft und der Rückstand in Methylenchlorid aufgenommen. Man schüttelt mehrmals mit Wasser aus, trocknet die organische Phase und erhält nach Chromatographie über SiO₂ 45 g (70 % d.Th.) der Phthalimidoverbindung vom Fp. 108 - 109°C.

### Beispiel 2

### 1-Benzyl-4-aminomethyl-pyrrolidin-2-on

a) 58 g (0,29 Mol) 1-Benzyl-4-nitrilo-pyrrolidin-2-on werden in Methanol gelöst und unter Zusatz von flüssigem Ammoniak über Raney-Nickel katalytisch hydriert. Nach dem Einengen der Reaktionslösung wird in Methanol gelöst, von Katalysatorresten abfiltriert und nach dem Erwärmen auf ca. 50°C mit 17 g Fumarsäure versetzt. Unter Rühren geht die Fumarsäure kurzzeitig in Lösung, danach beginnt die Kristallisation des 1-Benzyl-4-aminomethyl-pyrrolidin-2-on-fumarats.
   Ausbeute: 68 g (= 91 % d.Th.); Fp. 192-194°C.
b) Die Nitriloverbindung wird in 96 % Ausbeute aus dem entsprechenden Amid vom Fp. 162 - 166°C durch Dehydratisierung mittels POCl₃ in Dimethylformamid bei ca. 60°C als Öl erhalten.

### Beispiel 3

### Racematspaltung von 1-Benzyl-4-aminomethyl-pyrrolidin-2-on

a) 24,0 g (0,117 Mol) 1-Benzyl-4-aminomethyl-pyrrolidin-2-on werden in 200 ml heißem Methanol gelöst, 17,6 g (0,117 Mol) L(+)-Weinsäure werden ebenfalls in 200 ml heißem Methanol gelöst. Die beiden Lösungen werden zusammengegeben und unter Rühren auf Raumtemperatur abkühlen lassen, wobei das Salz auskristallisiert. Die Kristalle werden kalt abgesaugt, mit kaltem Methanol nachgewaschen und getrocknet.
   Ausbeute: 18,0 g 4-Aminomethyl-1-benzyl-pyrrolidin-2-on-tartrat, Fp. 204 - 206°C (aus Methanol), α_{D} = +6,3° (c = 1,0; Wasser).
b) Zur Umwandlung des Tartrats in die Base wird das Tartrat kalt in 20 ml Wasser und 10 ml konzentrierter Natronlauge gelöst, dreimal mit Methylenchlorid extrahiert, die vereinigten Methylenchloridphasen über MgSO₄ getrocknet und das Lösungsmittel i.V. abgezogen.
   Man erhält das (-)-4-Aminomethyl-1-benzyl-pyrrolidin-2-on, α_{D} = -8,4° (c = 1,0; Wasser).
c) Die bei der unter a) angegebenen Aufarbeitung anfallenden Mutterlaugen werden i.V. eingeengt. Man erhält 38,0 g des Tartrats, das kalt in 140 ml Wasser und 50 ml konzentrierter Natronlauge aufgenommen und dreimal mit Methylenchlorid ausgeschüttelt wird. Die vereinigten Methylenchloridphasen werden über MgSO₄ getrocknet und das Lösungsmittel i.V. abgezogen. Man erhält: 19,3 g Base, die, wie unter a) beschrieben, mit D-(-)-Weinsäure in das entsprechende Tartrat überführt wird. Ausbeute: 19,0 g vom Fp. 204 - 205°C.
d) Die Umwandlung des Tartrats in die Base erfolgt wie unter b) beschrieben.
   Man erhält 5,7 g (+)-4-Aminomethyl-1-benzyl-pyrrolidin-2-on mit einem Drehwert α_{D} = +8,4° (c = 1,0; Wasser).

### Beispiel 4

### (-)-1-Benzyl-4-dimethylaminomethyl-pyrrolidin-2-on

4,0 g (0,02 Mol) (-)-1-Benzyl-4-aminomethyl-pyrrolidin-2-on und 5,4 g 85%ige Ameisensäure werden mit 4,8 ml Formalinlösung versetzt und über Nacht bei 100°C (Ölbad) gerührt. Danach wird die überschüssige Säure i.V. abdestilliert, der Rückstand in Wasser aufgenommen, mit konzentrierter Natronlauge alkalisch gestellt und dreimal mit Methylenchlorid ausgeschüttelt. Die vereinigten Methylenchloridphasen werden mit Wasser gewaschen, über Natriumsulfat getrocknet, das Lösungsmittel i.V. eingeengt und der Rückstand über eine SiO₂-Säule filtriert (Fließmittel: Methylenchlorid: Methanol = 97:3). Die einheitliche Fraktion wird i.V. eingeengt.
Man erhält die Titelverbindung in einer Ausbeute von 3,5 g (als Öl).
α_{D} = -7,6° (c = 1,0; Methanol)
α_{D} = -16,8° (c = 1,0; Wasser).

Analog Beispiel 4 erhält man aus 5,8 g (0,028 Mol) (+)-1-Benzyl-4-aminomethyl-pyrrolidin-2-on, 7,9 g 85%iger Ameisensäure und 7 ml Formalinlösung 6,1 g (+)-1-Benzyl-4-dimethylaminomethyl-pyrrolidin-2-on, α_{D} = +7,9° (c = 1,0; Methanol).

### Beispiel 5

### 1-Benzyl-4-di-äthylaminomethyl-pyrrolidin-2-on

14 g (0,06 Mol) rohes 1-Benzyl-4-chlormethyl-pyrrolidin-2-on, hergestellt nach Beispiel 1a), 10 g Diäthylamin und 50 ml Dimethylformamid werden 2 Stunden bei 150°C im Autoklaven gerührt oder geschüttelt. Man dampft im Vakuum zur Trockne, nimmt den Rückstand in Methylenchlorid auf, wäscht zunächst mit Wasser und extrahiert anschließend die Titelverbindung mit zweimal 25 ml 2 N HCl. Die wäßrige Phase wird abgestrennt, mit Natronlauge alkalisch gestellt und die organische Base mit Methylenchlorid ausgeschüttelt. Die Methylenchloridphase wird eingedampft und der Rückstand im Vakuum destilliert.
Ausbeute: 10 g (61 % d.Th.) vom Kp_{0,05} = 155 - 158°C.

### Beispiel 6

### (-)-1-Benzyl-4-diäthylaminomethyl-pyrrolidin-2-on

11,5 g (0,056 Mol) (-)-1-Benzyl-4-aminomethyl-pyrrolidin-2-on, 130 ml Wasser, 13 g Acetaldehyd, 5,8 ml konzentrierte Salzsäure und 6,5 g Pd/C 20 % werden 5 1/4 Stunden bei 5 bar und 25°C hydriert. Der Rückstand wird eingedampft, in 30 ml Wasser aufgenommen und mit Methylenchlorid ausgeschüttelt. Die salzsaure, wäßrige Lösung wird alkalisch gestellt und ebenfalls mit Methylenchlorid extrahiert.
Durch Destillation im Kugelrohr erhält man 11,2 g (76,4 % d.Th.) der Titelverbindung α_{D} = -9,4 (c = 1,0; Methanol).

Analog Beispiel 6 erhält man durch Hydrierung von 8,4 g (0,041 Mol) (+)-1-Benzyl-4-aminomethyl-pyrrolidin-2-on, 95 ml Wasser, 9,5 Acetaldehyd, 4,2 ml konzentrierte Salzsäure und 4,7 g Pd/C 20 % das (+)-1-Benzyl-4-diäthylaminomethyl-pyrrolidin-2-on, α_{D} = +9,4 (c = 1,0; Methanol).

### Beispiel 7

### 1-(4-Fluor-benzyl)-4-N-methylpiperazinylmethyl-pyrrolidin-2-on

a) 24 g (0,11 Mol) 1-(4-Fluor-benzyl)-4-hydroxymethyl-pyrrolidin-2-on werden mit 10 ml (0,14 Mol) Thionylchlorid in 200 ml Methylenchlorid zunächst 10 Stunden und nach Zugabe von weiteren 10 ml Thionylchlorid weitere 6 Stunden unter Rückfluß gekocht. Unter Eiskühlung wird mit Ammoniak neutralisiert und nach dem Abtrennen der organischen Phase diese getrocknet und eingedampft. Es hinterbleiben 23 g (92 % d.Th.) eines rotbraunen Öls, das ohne weitere Reinigung verwendet wird.
b) 5 g (0,002 Mol) des obigen Öls werden mit 4,4 g (0,04 Mol) 1-Methyl-piperazin in 30 ml Dimethylformamid 1 - 2 Stunden unter Rückfluß gekocht. Man destilliert anschließend das Dimethylformamid weitgehend im Vakuum ab, nimmt den Rückstand in Methylenchlorid auf, wäscht mit Wasser, trocknet die organische Phase und dampft erneut ein. Der Rückstand wird über SiO₂ mit Methylenchlorid/Methanol 95:5 als Fließmittel chromatographiert. Die Hauptfraktion wird eingedampft und der Rückstand (5 g) in 30 ml Methanol gelöst. Zu dieser Lösung werden 2,8 g Fumarsäure gegeben. 5,2 g (48 % d.Th.) der Titelverbindung scheiden sich kristallin als Fumarat ab.
   Fp. 179 - 180°C.

### Beispiel 8

### 1-(4-Fluorbenzyl)-4-morpholinomethyl-pyrrolidin-2-on

a) 8,9 g (0,04 Mol) 1-(4-Fluorbenzyl)-4-hydroxymethylpyrrolidin-2-on in 100 ml absolutem Methylenchorid und 4,8 g Pyridin werden mit 6,9 g (0,06 Mol) Methansulfonsäurechlorid versetzt. Man kocht 2,5 Stunden unter Rückfluß, kühlt ab und schüttelt mit verdünntem Ammoniak und Wasser. Die organische Phase wird getrocknet und eingedampft
   Man erhält 11 g (93 % d.Th.) rohen Ester vom Fp. 84 - 86°C.
b) 6,7 g (0,023 Mol) Ester und 2,6 g (0,03 Mol) Morpholin werden in 20 ml Dioxan 2 Stunden unter Rückfluß gekocht. Man dampft das Lösungsmittel im Vakuum ab, nimmt den Rückstand in Methylenchlorid auf und extrahiert mit 50 ml 2 n Salzsäure. Die wäßrigen Auszüge werden mit Ammoniak alkalisch gestellt und die ölige Base mit Methylenchlorid ausgeschüttelt. Die Methylenchloridphase wird getrocknet und eingedampft. Den Rückstand (4,2 g) nimmt man in 30 ml Methanol aus und fügt in der Wärme 1,2 g Fumarsäure hinzu. Nach dem Abkühlen fällt das Fumarat der Titelverbindung kristallin aus.
   Ausbeute: 7 g = 57 % d.Th. farblose Kristalle vom Fp. 175 - 176°C.

### Beispiel 9

### 1-(4-Fluorbenzyl)-4-amino-pyrrolidin-2-on

a) 4,0 g (0,013 Mol) Mesyl-Ester, hergestellt nach Beispiel 7, werden mit 2,8 g (0,015 Mol) Phthalimidkalium in 50 ml Dimethylformamid 30 Minuten unter Rückfluß gekocht. Man dampft im Vakuum ein, nimmt den Rückstand in Methylenchlorid auf, wäscht mit Wasser, trocknet die organische Pase und dampft erneut ein. Der Rückstand wird mit Äther verrieben und ergibt 3,6 g (78 % d.Th.) hellgraue Kristalle vom Fp. 124 - 125°C.
b) 3,5 g (0,1 Mol) obiger Phthalimidverbindung werden zusammen mit 5,5 g Hydrazinhydrat in 200 ml Alkohol 4-Stunden bei Raumptemperatur gerührt. Die Aufarbeitung erfolgt wie in Beispiel 2 beschrieben. Es werden 2,5 g (89 % d.Th.) Fumarat der Titelverbindung vom Fp. 214 - 215°C erhalten.

Die Titelverbindung kann auch erhalten werden, indem man 5 g (16 mMol) Mesyl-Ester (siehe Beispiel 7) in 100 ml Dimethylformamid löst und nach Zugabe von 1,3 g Natriumazid 2 Stunden auf 100° C erwärmt, das nach entsprechender Aufarbeitung erhaltene Öl in Methanol mit Raney-Nickel hydriert und die Base wie oben in das Fumarat umwandelt. Ausbeute: 4,2 g (90 % d.Th.)

Analog der in den vorstehenden Beispielen beschriebenen Arbeitsweise wurden ferner die folgenden Endprodukte erhalten:

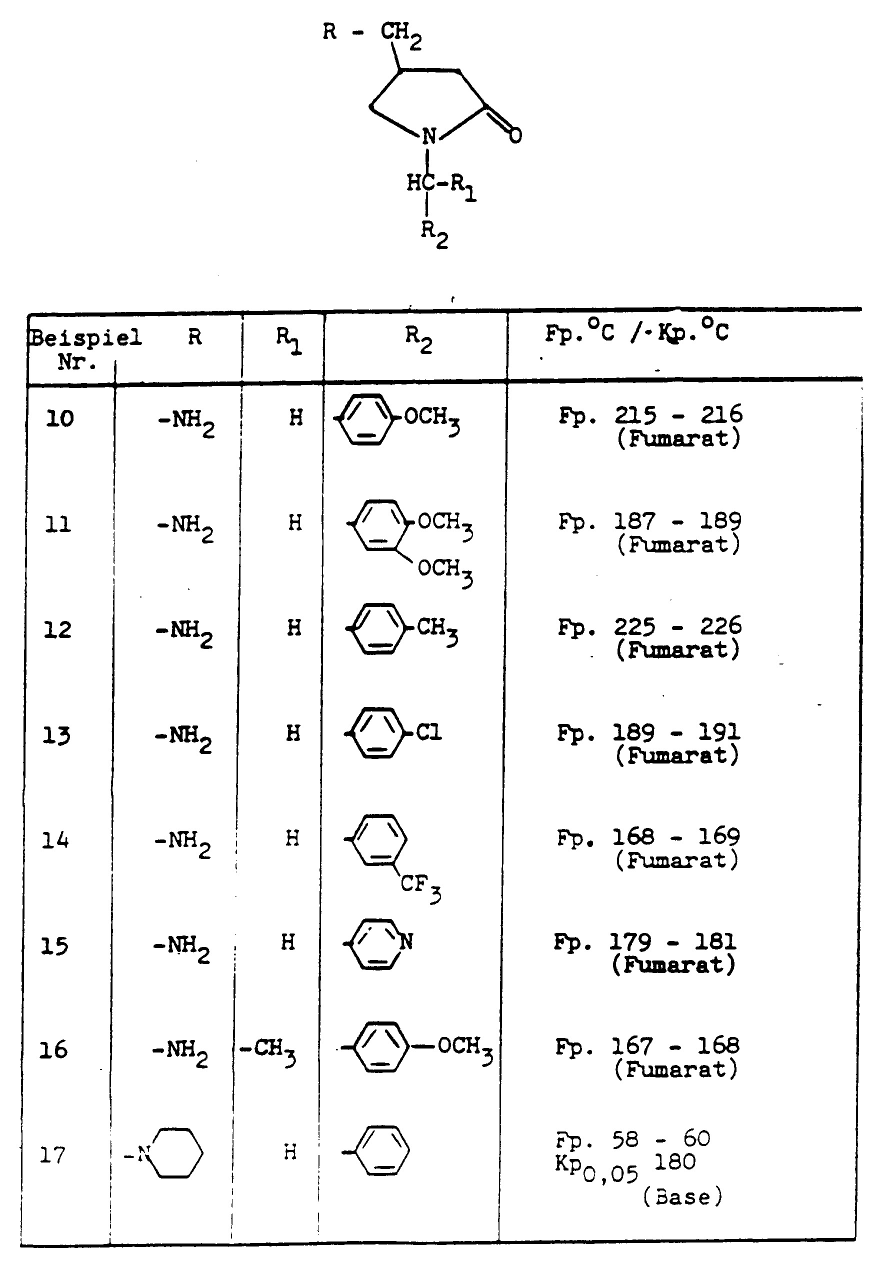

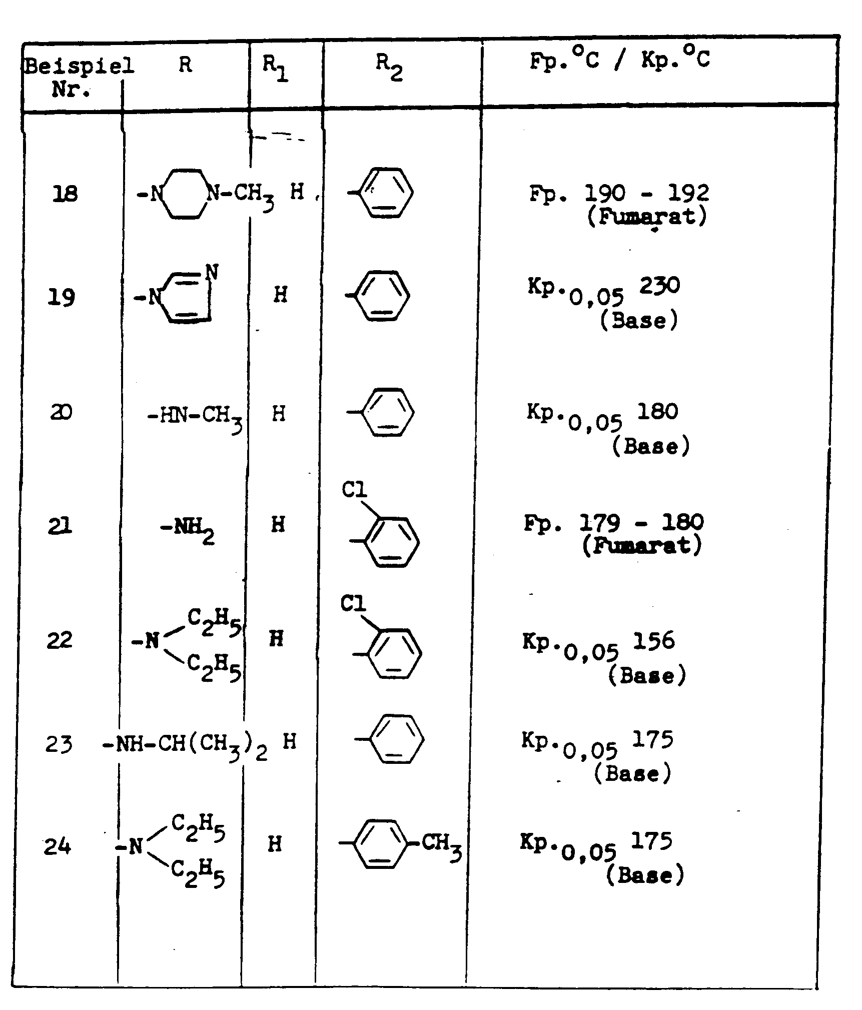

### Beispiel 25

### 1-Benzyl-5-dimethylaminomethyl-pyrrolidin-2-on

a) Eine Lösung von 10,26 g (0,05 Mol) 1-Benzyl-5-hydroxymethyl-pyrrolidin-2-on (Fp. 76 - 77°C) und 5,6 g (0,055 Mol) Triäthylamin in 80 ml Methylenchlorid wird mit 6,3 g (0,055 Mol) Methansulfonsäurechlorid in 20 ml Methylenchlorid versetzt. Das Reaktionsgemisch wird anschließend 1 Stunde unter Rückfluß erhitzt und nach dem Abkühlen mit Wasser ausgeschüttelt. Die organische Phase wird nach dem Trocknen über wasserfreiem Natriumsulfat am Rotationsverdampfer eingedampft. Man erhält 14.1 g (gelbes Öl) rohen 1-Benzyl-5-hydroxymethyl-pyrrolidin-2-on-methansulfonsäureester, der ohne weitere Reinigung in die nächste Reaktionsstufe eingesetzt wird.
b) 8,5 g (0,03 Mol) des nach a) erhaltenen Mesylats werden mit einer Lösung von 10 g Dimethylamin in 60 ml Dioxan in einem Autoklaven 3 Stunden auf 150°C erhitzt. Die abgekühlte Reaktionsmischung wird im Vakuum zur Trockne eingeengt. Man löst den Rückstand in 2 n Salzsäure und extrahiert mit Äther. Die saure wäßrige Phase wird mit konzentriertem Ammoniak alkalisch gestellt und mit Methylenchlorid ausgeschüttelt. Die Methylenchloridlösung wird getrocknet und eingedampft. Der Rückstand (6,5 g) wird mit einer äquivalenten Menge Fumarsäure in das saure Fumarat der Titelverbindung überführt.
   Ausbeute: 6,4 g (61 % d.Th.); Fp. 137 - 138°C.

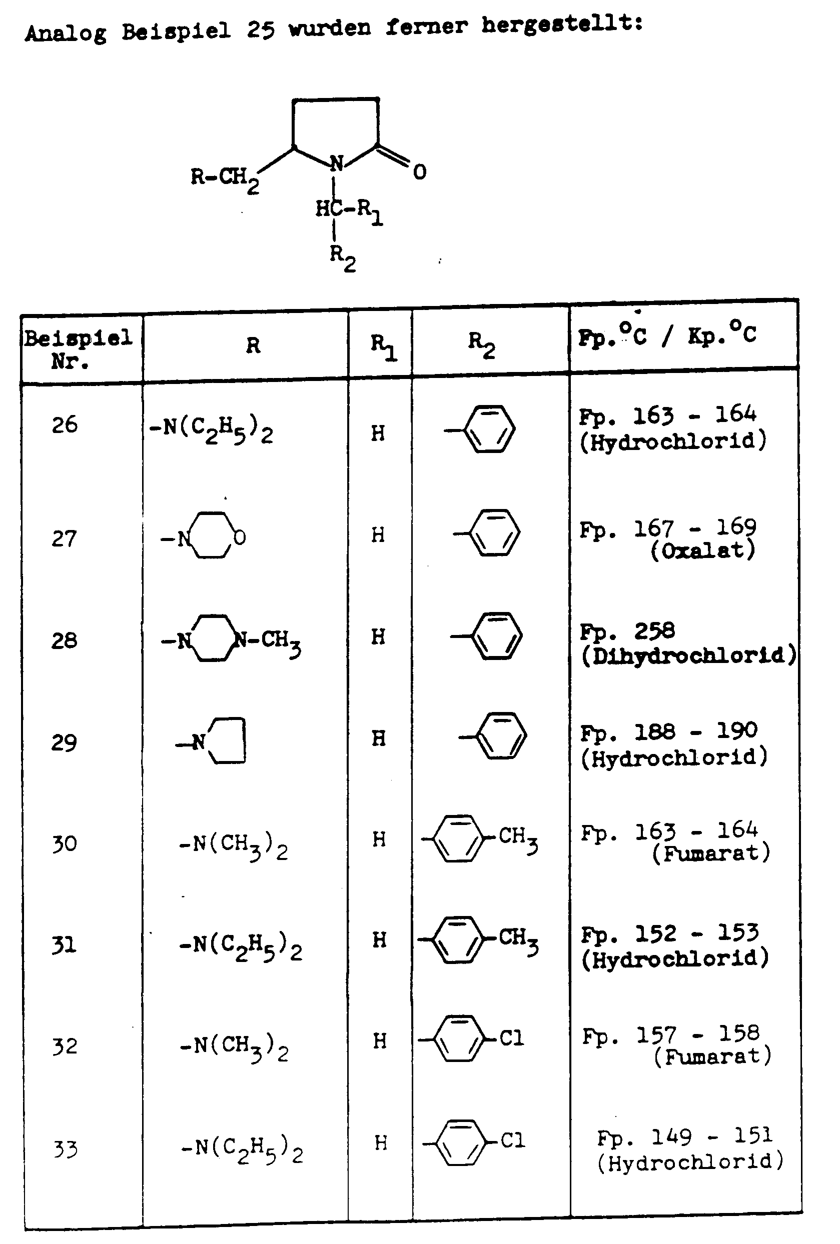

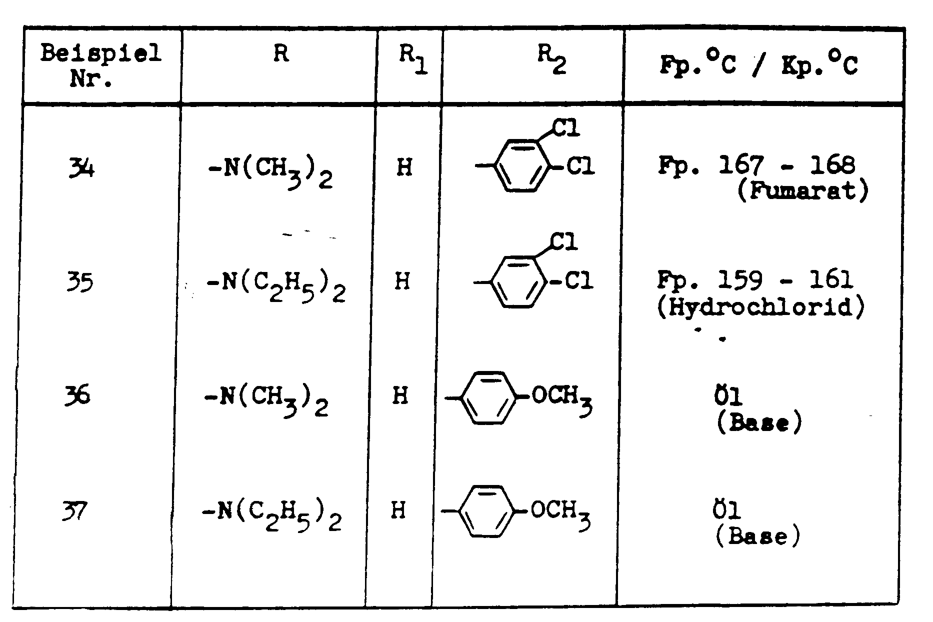

### Beispiel 38

### 1-Benzyl-5-aminomethyl-pyrrolidin-2-on

16,4 g (0,07 Mol) 1-Benzyl-5-hydroxymethyl-pyrrolidin-2-on-methansulfonsäureester (siehe Beispiel 25 a)) werden in 200 ml Dimethylformamid gelöst und nach Zugabe von 4,6 g (0,07 Mol) Natriumazid 90 Minuten bei 100°C gerührt. Nach Eindampfen, Verteilen zwischen Wasser und Methylenchlorid und Aufarbeiten der organischen Phase erhält man 13,8 g (92 % d.Th.) Öl, das roh weiter umgesetzt werden kann. Es wird in 200 ml Methanol gelöst und nach Zugabe von Raney-Nickel bei 20°C und 5 bar hydriert. Nach Absaugen des Katalysators und Eindampfen des Filtrats werden 11 g (85 % d.Th.) Öl erhalten, die gelöst in Methanol und nach Zugabe von Fumarsäure das gewünschte Hemifumarat der Titelverbindung ergeben (Fp. 187 - 188°C).

### Pharmazeutische Formulierungsbeispiele

| A) Tabletten | pro Tablette |
|---|---|
| Wirkstoff | 100 mg |
| Milchzucker (pulverisiert) | 140 mg |
| Maisstärke | 240 mg |
| Polyvinylpyrrolidon | 15 mg |
| Magnesiumstearat | 5 mg |
| | 5̅0̅0̅ m̅g̅ |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) Tabletten | pro Tablette |
|---|---|
| Wirkstoff | 80 mg |
| Maisstärke | 190 mg |
| Milchzucker | 55 mg |
| Mikrokristalline Cellulose | 35 mg |
| Polyvinylpyrrolidon | 15 mg |
| Natrium-carboxxymethylstärke | 23 mg |
| Magnesiumstearat | 2 mg |
| | 4̅0̅0̅ m̅g̅ |

Der feingemahlene Wirkstoff, ein Teil der Maisstärke, Milchzucker, mikrokristalline Cellulose und Polyvinylpvrrolidon werden miteinander vermischt, die Mischung gesiebt und mit dem Rest der Maisstärke und Wasser zu einem Granulat verarbeitet, welches getrocknet und gesiebt wird. Dazu gibt man die Natrium-carboxymethylstärke und das Magnesiumstearat, vermischt und verpreßt das Gemisch zu Tabletten geeigneter Größe.

### C) Ampullen

1-Benzyl-4-aminomethyl-pyrrolidin-2-on-Fumarat 50,0 mg
Natriumchlorid 10,0 mg
bidestilliertes Wasser q.s. ad 1,0 ml

### Herstellung:

Der Wirkstoff und das Natriumchlorid werden in bidestilliertem Wasser gelöst und die Lösung in Ampullen steril abgefüllt.

### D) Tropfen

1-Benzyl-4-aminomethyl-pyrrolidin-2-on-Fumarat 5,0 mg
p-Hydroxybenzoesäuremethylester 0,1 g
p-Hydroxybenzoesäuremethylester 0,1 g
entmineralisiertes Wasser q.s. ad 100,0 ml

### Herstellung:

Der Wirkstoff und die Konservierungsmittel werden in demineralisiertem Wasser gelöst und die Lösung filtriert und in Flaschen zu je 100 ml abgefüllt.

## Patentansprüche

1. Verwendung von Verbindungen der allgemeinen Formel I 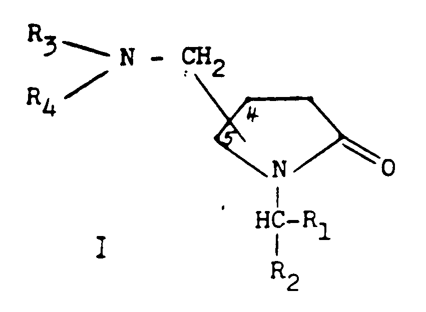 worin
R₁ Wasserstoff oder einen Alkylrest,
R₂ einen Phenylrest, der ein- oder zweifach durch Alkoxy, Fluor, Chlor, Brom, Trifluormethyl, Alkyl, Hydroxy oder Nitro substituiert sein kann oder einen Pyridylrest,
R₃ Wasserstoff oder einen Alkylrest und
R₄ Wasserstoff oder einen Alkylrest bedeuten können oder beide Reste R₃ und R₄ zusammen mit dem Stickstoffatom einen gesättigten 5- oder 6-Ring, der als weiteres Heteroatom ein O- oder N-Atom enthalten kann und gegebenenfalls durch Methyl substituiert sein kann oder einen Imidazolring bilden und wobei die Aminoalkylgruppe in 4- oder 5-Stellung steht und deren pharmakologisch verträgliche Säureadditonssalze zur Herstellung eines Arzneimittels zur Behandlung von Depressionen.

2. Verwendung von Verbindungen der allgemeinen Formel I 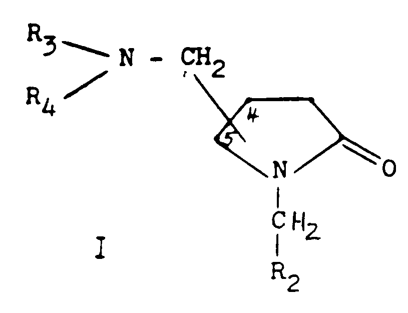
R₂ einen Phenylrest, der ein- oder zweifach durch Alkoxy, Fluor, Chlor, Brom, Trifluormethyl, Alkyl, Hydroxy oder Nitro substituiert sein kann oder einen Pyridylrest,
R₃ Wasserstoff oder einen Alkylrest mit 1 bis 2 Kohlenstoffatomen und
R₄ Wasserstoff oder einen Alkylrest mit 1 bis 2 Kohlenstoffatomen bedeuten können oder beide Reste R₃ und R₄ zusammen mit dem Stickstoffatom einen gesättigten 5- oder 6-Ring, wobei der 6-Ring als weiteres Heteroatom ein O-oder N-Atom enthalten kann und gegebenenfalls durch Methyl substituiert sein kann, bilden und wobei die Aminoalkylgruppe, in 4- oder 5-Stellung steht und deren pharmakologisch verträgliche Säureadditonssalze zur Herstellung eines Arzneimittels zur Behandlung von Depressionen.

3. Verwendung von 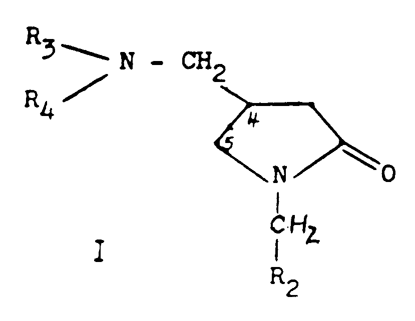 Verbindungen der allgemeinen Formel I
R₂ einen Phenylrest, der einfach durch Alkoxy, Fluor, Chlor, Brom, Trifluormethyl, Alkyl, Hydroxy oder Nitro substituiert sein kann oder einen Pyridylrest,
R₃ Wasserstoff oder einen Alkylrest mit 1 bis 2 Kohlenstoffatomen und
R₄ Wasserstoff oder einen Alkylrest mit 1 bis 2 Kohlenstoffatomen bedeuten können oder beide Reste R₃ und R₄ zusammen mit dem Stickstoffatom einen gesättigten 5- oder 6-Ring, wobei der 6-Ring als weiteres Heteroatom ein O-oder N-Atom enthalten kann und gegebenenfalls durch Methyl substituiert sein kann und deren pharmakologisch verträgliche Säureadditonssalze zur Herstellung eines Arzneimittels zur Behandlung von Depressionen.

4. Verwendung von Verbindungen der allgemeinen Formel I 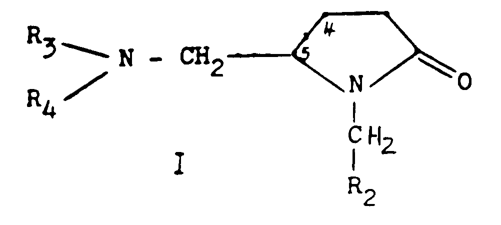
R₂ einen Phenylrest, der einfach durch Alkoxy, Fluor, Chlor, Brom, Trifluormethyl, Alkyl, Hydroxy oder Nitro substituiert sein kann oder einen Pyridylrest,
R₃ Wasserstoff oder einen Alkylrest mit 1 bis 2 Kohlenstoffatomen und
R₄ Wasserstoff oder einen Alkylrest mit 1 bis 2 Kohlenstoffatomen bedeuten können oder beide Reste R₃ und R₄ zusammen mit dem Stickstoffatom einen gesättigten 5- oder 6-Ring, wobei der 6-Ring als weiteres Heteroatom ein O- oder N-Atom enthalten kann und gegebenenfalls durch Methyl substituiert sein kann und deren pharmakologisch verträgliche Säureadditonssalze zur Herstellung eines Arzneimittels zur Behandlung von Depressionen.

5. Verwendung von 1-Benzyl-4-aminomethyl-pyrrolidin-2-on sowie gegebenenfalls seiner pharmakologisch verträglichen Säureadditionssalze zur Herstellung eines Arzneimittels zur Behandlung von Depressionen.

6. Verwendung von 1-Benzyl-5-N,N-dimethylaminomethyl-pyrrolidin-2-on sowie seiner pharmakologisch verträglichen Säureadditonssalze zur Herstellung eines Arzneimittels zur Behandlung von Depressionen.

## Claims

1. Use of compounds of general formula I 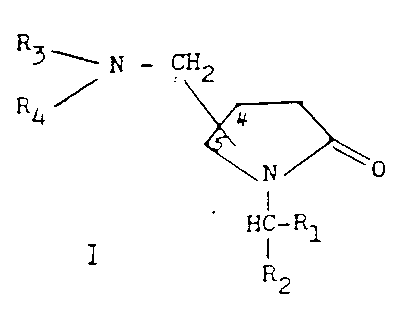 wherein
R₁ represents hydrogen or an alkyl group,
R₂ represents a phenyl group which may be mono- or disubstituted by alkoxy, fluorine, chlorine, bromine, trifluoromethyl, alkyl, hydroxy or nitro, or R₂ represents a pyridyl group,
R₃ represents hydrogen or an alkyl group and
R₄ may represent hydrogen or an alkyl group or the two groups R₃ and R₄ together with the nitrogen atom may represent a saturated 5- or 6-membered ring which may contain an O or N atom as a further heteroatom and may optionally be substituted by methyl or they may form an imidazole ring, whilst the aminoalkyl group is in the 4 or 5 position, and the pharmacologically acceptable acid addition salts thereof,
for the preparation of a pharmaceutical composition for treating depression.

2. Use of compounds of general formula I 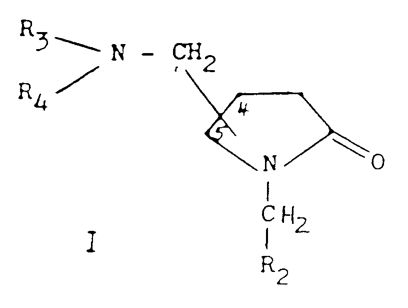 wherein
R₂ represents a phenyl group which may be mono- or disubstituted by alkoxy, fluorine, chlorine, bromine, trifluoromethyl, alkyl, hydroxy or nitro, or R₂ represents a pyridyl group,
R₃ represents hydrogen or an alkyl group having 1 to 2 carbon atoms and
R₄ may represent hydrogen or an alkyl group having 1 to 2 carbon atoms or the two groups R₃ and R₄ together with the nitrogen atom may represent a saturated 5- or 6-membered ring which may contain an O or N atom as a further heteroatom and may optionally be substituted by methyl, whilst the aminoalkyl group is in the 4 or 5 position, and the pharmacologically acceptable acid addition salts thereof,
for preparing a pharmaceutical composition for the treatment of depression.

3. Use of compounds of general formula I 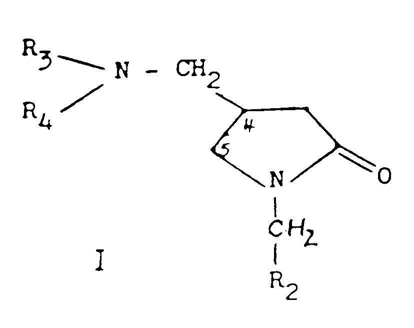 wherein
R₂ represents a phenyl group which may be mono-substituted by alkoxy, fluorine, chlorine, bromine, trifluoromethyl, alkyl, hydroxy or nitro, or R₂ represents a pyridyl group,
R₃ represents hydrogen or an alkyl group having 1 to 2 carbon atoms and
R₄ may represent hydrogen or an alkyl group having 1 to 2 carbon atoms or the two groups R₃ and R₄ together with the nitrogen atom may represent a saturated 5- or 6-membered ring which may contain an O or N atom as a further heteroatom and may optionally be substituted by methyl, whilst the aminoalkyl group is in the 4 or 5 position, and the pharmacologically acceptable acid addition salts thereof,
for preparing a pharmaceutical composition for the treatment of depression.

4. Use of compounds of general formula I 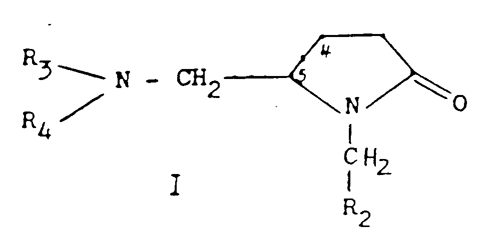 wherein
R₂ represents a phenyl group which may be mono-substituted by alkoxy, fluorine, chlorine, bromine, trifluoromethyl, alkyl, hydroxy or nitro, or R₂ represents a pyridyl group,
R₃ represents hydrogen or an alkyl group having 1 to 2 carbon atoms and
R₄ may represent hydrogen or an alkyl group having 1 to 2 carbon atoms or the two groups R₃ and R₄ together with the nitrogen atom may represent a saturated 5- or 6-membered ring which may contain an O or N atom as a further heteroatom and may optionally be substituted by methyl, whilst the aminoalkyl group is in the 4 or 5 position, and the pharmacologically acceptable acid addition salts thereof,
for preparing a pharmaceutical composition for the treatment of depression.

5. Use of 1-benzyl-4-aminomethyl-pyrrolidin-2-one and optionally the pharmacologically acceptable acid addition salts thereof for preparing a pharmaceutical composition for the treatment of depression.

6. Use of 1-benzyl-5-N,N-dimethylaminomethyl-pyrrolidin-2-one and the pharmacologically acceptable acid addition salts thereof for preparing a pharmaceutical composition for the treatment of depression.

## Revendications

1. Utilisation de composés de formule générale I 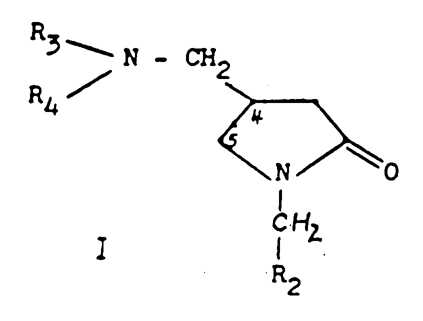 dans laquelle
R₁ peut représenter un atome d'hydrogène ou un reste alkyle,
R₂ peut représenter un reste phényle, qui peut être substitué une ou deux fois par alcoxy, fluor, chlore, brome, trifluorométhyle, alkyle, hydroxy ou nitro, ou un reste pyridyle,
R₃ peut représenter un atome d'hydrogène ou un reste alkyle et
R₄ peut représenter un atome d'hydrogène ou un reste alkyle, ou bien les deux restes R₃ et R₄ forment avec l'atome d'azote un cycle saturé à 5 ou 6 chaînons qui peut contenir comme autre hétéroatome un atome d'oxygène ou d'azote et qui peut éventuellement être substitué par méthyle, ou bien ils forment un cycle imidazole, et dans laquelle le groupe aminoalkyle est situé en position 4 ou 5, et de leurs sels d'addition d'acide compatibles du point de vue pharmacologique pour la préparation d'un médicament pour le traitement des dépressions.

2. Utilisation de composés de formule générale I 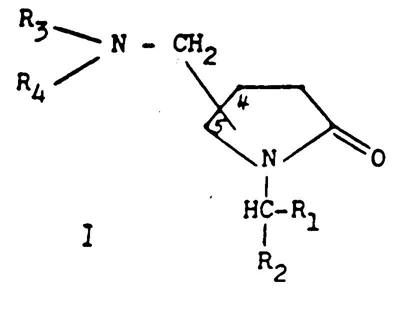 dans laquelle
R₂ peut représenter un reste phényle, qui peut être substitué une ou deux fois par alcoxy, fluor, chlore, brome, trifluorométhyle, alkyle, hydroxy ou nitro, ou un reste pyridyle,
R₃ peut représenter un atome d'hydrogène ou un reste alkyle contenant 1 à 2 atomes de carbone et
R₄ peut représenter un atome d'hydrogène ou un reste alkyle contenant 1 à 2 atomes de carbone, ou bien les deux restes R₃ et R₄ forment avec l'atome d'azote un cycle saturé à 5 ou 6 chaînons, le cycle à 6 chaînons pouvant contenir comme autre hétéroatome un atome d'oxygène ou d'azote et pouvant éventuellement être substitué par méthyle, et dans laquelle le groupe aminoalkyle est situé en position 4 ou 5, et de leurs sels d'addition d'acide compatibles du point de vue pharmacologique pour la préparation d'un médicament pour le traitement des dépressions.

3. Utilisation de composés de formule générale I 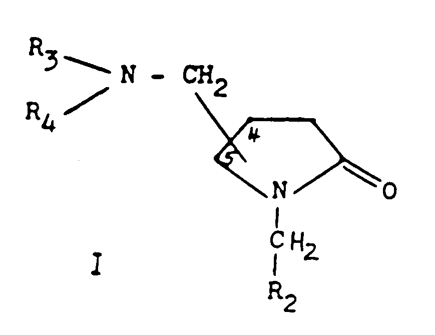 dans laquelle
R₂ peut représenter un reste phényle, qui peut être substitué une fois par alcoxy, fluor, chlore, brome, trifluorométhyle, alkyle, hydroxy ou nitro, ou un reste pyridyle,
R₃ peut représenter un atome d'hydrogène ou un reste alkyle contenant 1 à 2 atomes de carbone et
R₄ peut représenter un atome d'hydrogène ou un reste alkyle contenant 1 à 2 atomes de carbone, ou bien les deux restes R₃ et R₄ forment avec l'atome d'azote un cycle saturé à 5 ou 6 chaînons, le cycle à six chaînons pouvant contenir comme autre hétéroatome un atome d'oxygène ou d'azote et pouvant éventuellement être substitué par méthyle, et de leurs sels d'addition d'acide compatibles du point de vue pharmacologique pour la préparation d'un médicament pour le traitement des dépressions.

4. Utilisation de composés de formule générale I 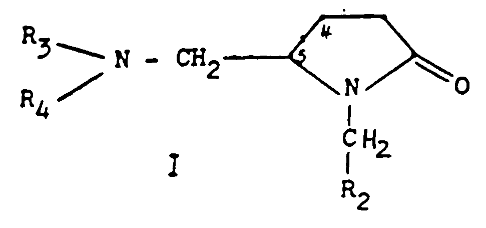 dans laquelle
R₂ peut représenter un reste phényle, qui peut être substitué une fois par alcoxy, fluor, chlore, brome, trifluorométhyle, alkyle, hydroxy ou nitro, ou un reste pyridyle,
R₃ peut représenter un atome d'hydrogène ou un reste alkyle contenant 1 à 2 atomes de carbone et
R₄ peut représenter un atome d'hydrogène ou un reste alkyle contenant 1 à 2 atomes de carbone, ou bien les deux restes R₃ et R₄ forment avec l'atome d'azote un cycle saturé à 5 ou 6 chaînons, le cycle à six chaînons pouvant contenir comme autre hétéroatome un atome d'oxygène ou d'azote et pouvant éventuellement être substitué par méthyle, et de leurs sels d'addition d'acide compatibles du point de vue pharmacologique pour la préparation d'un médicament pour le traitement des dépressions.

5. Utilisation de la benzyl-1-aminométhyl-4-pyrrolidinone-2 et éventuellement de ses sels d'addition d'acide compatibles du point de vue pharmacologique pour la préparation d'un médicament pour le traitement des dépressions.

6. Utilisation de la benzyl-1-diméthyl-N,N-aminométhyl-5-pyrrolidinone- 2 et de ses sels d'addition d'acide compatibles du point de vue pharmacologique pour la préparation d'un médicament pour le traitement des dépressions.
